# EUROPEAN PATENT SPECIFICATION

(11) **EP 1 165 002 B1**
(45) Date of publication and mention of the grant of the patent: **04.12.2002**
(21) Application number: 00905508.8
(22) Date of filing: 19.01.2000
(51) Int. Cl.: A61F 13/475

(54) **ABSORBENT ARTICLE WITH IMPROVED LEAKTIGHTNESS AND IMPROVED FIT**
ABSORBIERENDE ARTIKEL MIT VERBESSERTEM AUSLAUFSCHUTZ UND VERBESSERTER PASSFORM
ARTICLE ABSORBANT A ETANCHEITE AMELIOREE ET EPOUSANT MIEUX LES FORMES

(30) Priority: 29.01.1999 SE 9900288
(43) Date of publication of application: 02.01.2002
(73) Proprietor: SCA Hygiene Products AB, 405 03 Göteborg (SE)
(72) Inventor: HERMANSSON, Kent, S-426 54 Västra Frölunda (SE); JOHANSSON, Asa, S-417 16 Göteborg (SE); RAGNARSSON, Christina, S-412 69 Göteborg (SE); ELFSTRÖM, Anna-Karin, S-423 38 Torslanda (SE)
(74) Representative: Olsson, Stefan
(86) International application number: SE0000094
(87) International publication number: WO00044326

(56) References cited:
- EP-A1- 0 606 082
- WO-A1-95/31164
- WO-A1-97/17921
- GB-A- 2 270 247
- SE-C2- 501 010
- SE-C2- 508 538
- US-A- 5 779 689

## Description

### TECHNICAL FIELD:

The invention relates to an absorbent article having a longitudinal direction and a transverse direction, two side edges running in the longitudinal direction, and a first end edge running in the transverse direction and a second end edge running in the transverse direction, and comprising a liquid-tight cover sheet, a liquid-permeable cover sheet, an absorption body enclosed between the two cover sheets, and elastic members which run in an arc shape across the article between two opposite edges.

### BACKGROUND:

When designing absorbent articles such as diapers and incontinence pads, it is above all important to ensure that the article does not leak when it is being used. The leaktightness is of course dependent on the article having sufficient absorption capacity. It is also particularly important for the article to be designed such that excreted body matter is collected and passed into the article. A problem encountered in connection with absorbent articles of this type is in fact that liquid runs across the surface of the article and leaks out from the article before the liquid has had time to be absorbed by the article. Such leakage can occur, for example, when large amounts of liquid impact the article within a short space of time, or when liquid is delivered towards the article at high pressure.

A common measure for reducing the risk of leakage of an absorbent article such as a diaper or an incontinence pad is to provide the article with elastic members. The elastic members can be used to give the absorbent article an improved fit, so that the article closely matches the anatomy of the user. In addition, elastic members can additionally be used to create different types of leakage barriers. For example, it is customary to arrange elastic members along the side edges of an absorbent article, as a result of which the side edges are drawn together and form leakage barriers. In absorbent articles which are shaped like pants or which during use are joined together to form the shape of pants, elastic members arranged along the side edges are used to form elastic leg bands which, during use, hold the absorbent article tightly around the user's legs.

EP 760 643 and WO 99/16398, for example, describe absorbent articles in which elastic members arranged in a curved pattern across the surface of the article are used to create a cup-shaped liquid-receiving area. In WO 9531164 is disclosed an absorbent article, having a longitudinal extension along, and being symmetric about a longitudinal axis and comprising elastic means extending along said longitudinal extension and defining an outwardly convex curved path on either side of said longitudinal axis, said elastic means being attached to the topsheet contacting the absorbent core. The purpose with the elastic means is to create an absorbent article with improved protection against lateral leakage. Although the elastic members, described in said publications, do considerably increase the leaktightness of an absorbent article, further improvements are still needed.

It has in particular proven difficult to obtain sufficient leaktightness and a good fit in absorbent articles of the type which are arranged under a pair of underpants and which, during use, are held in place by the underpants alone. Such an absorbent article lacks the sealing leg elastics which have been described above. In addition, there is a risk of the article moving in the underpants during use and thus taking up an incorrect position which, on the one hand, can give rise to leakage and, on the other hand, can result in the article being uncomfortable to wear. Moreover, the fact that the article is not held securely in a defined position means that the risk of deformation of the article is greater than in the case of one shaped like underpants. For example, the article may become folded between the user's legs during use, as a result of which the surface available for receiving liquid becomes inadequate and/or undesired creases are formed in which liquid can run out from the article. In addition, the article's fit and the user's comfort are negatively affected if the article is deformed in an uncontrolled manner during use.

### OBJECT OF THE INVENTION:

One aim of the invention is to make available an absorbent article with improved leaktightness and improved fit. The invention provides in particular improved leaktightness and improved fit for absorbent articles of the type supported by a separate pair of underpants. A further aim of the invention is to provide an absorbent article or insert which has less tendency to move or become deformed in an uncontrolled manner during use.

### DESCRIPTION OF THE INVENTION:

An article designed according to the invention is chiefly distinguished in that a first elastic member is secured to the liquid-permeable cover sheet and forms a first arc-shaped curve which runs in across the absorption body and is directed towards a first edge of the article, and in that a second elastic member is secured to the liquid-tight cover sheet and forms a second arc-shaped curve which runs in across the absorption body, and is directed towards said first edge, the second elastic member being arranged between the first elastic member and the said first edge.

The first, or inner, arc-shaped curve and the second, or outer, arc-shaped curve can advantageously be uniform.

The distance between the first elastic member and the second elastic member is suitably from 5 millimetres to 75 millimetres, preferably from 10 millimetres to 35 millimetres.

Each elastic member can comprise one or more part members, such as threads, bands, filaments or the like.

According to one embodiment, the invention is distinguished in that hat the said first and second elastic members are arranged at the front portion and run in the transverse direction of the article between the side edges of the article, the arc-shaped curve being directed towards a first end edge, in that a third elastic member is secured to the liquid-permeable cover sheet and forms a third arc-shaped curve which is directed towards the second end edge of the article, and in that a fourth elastic member is secured to the liquid-tight cover sheet and forms a fourth arc-shaped curve, said third and fourth elastic members are arranged at the rear portion, the fourth elastic member being arranged between the third elastic member and the second end edge of the article.

An article designed according to the invention can thus be provided with arc-shaped elastic members arranged in pairs only at one end portion. However, it is advantageous, in a design with transverse elastic members, to arrange arc-shaped elastic members at both end portions, since in this way it is possible to create a liquid-receiving cup, enclosed by elastic members, at the crotch area of the article. Crotch area signifies that part of the article which, during use, is intended to be arranged between the user's legs and which constitutes the receiving area for most of the excreted body fluid passed to the article.

It may also be appropriate, in a design with transverse elastic members, to provide the absorbent article with elastic members arranged along the side edges of the article in order to form leakage barriers and to facilitate the shaping of the article in the longitudinal direction. Elastic members arranged along the side edges additionally cooperate with the curved elastic members at the front and/or rear portions of the article so as to form an area enclosed by elastic members.

According to another embodiment, the invention is distinguished in that the said first and second elastic members run in the longitudinal direction of the article between the end edges of the article, the first and second arc-shaped curves being directed towards a first side edge in that a third elastic member is secured to the liquid-permeable cover sheet and forms a third arc-shaped curve which is directed towards the second side edge of the article, said third elastic member is arranged symmetrical with the first longitudinal elastic member on the opposite side of the longitudinal centre line of the article, and in that a fourth elastic member is secured to the liquid-tight cover sheet and forms a fourth arc-shaped curve, the fourth elastic member being arranged between the third elastic member and the second side edge of the article.

A number of advantages are achieved by combining a first, inner elastic member, which is arranged on that surface of the absorbent article intended to face towards the user when the article is being used, and a second outer elastic member which is arranged on that surface of the absorbent article intended to face away from the user during use.

The inner elastic member forms an essentially transverse or longitudinal, raised and curved barrier which prevents liquid from flowing freely across the surface of the article. In addition, the inner elastic member is kept in tight contact with the user's body during use and counteracts the development of play between the article and the user's body.

The main function of the outer elastic member is to curve the absorbent article so that it better matches the shape of the user's body. The outer elastic member additionally strengthens the barrier effect of the inner elastic member and cooperates with the inner elastic member to create a cup-shaped area on that surface of the article which faces towards the user during use. In addition, the outer elastic member gives rise in a controlled manner to fold formations in the liquid-tight cover sheet, which is advantageous because such fold formations increase the friction between the absorbent article and the user's underwear.

By arranging the outer elastic member on the outside of the liquid-tight sheet, i.e. that side of the sheet which during use of the article faces towards the user's underwear, the friction can be further increased. This reduces the risk of the absorbent article moving to an incorrect position in the underwear.

### BRIEF DESCRIPTION OF THE FIGURES:

The invention will be described in greater detail below with reference to the figures which are shown on the attached drawings, and of which:
Figure 1 shows a first embodiment of an incontinence diaper according to the invention,
Figure 2 shows a longitudinal section, along the line II-II, through the diaper in Figure 1,
Figure 3 shows a second embodiment of an incontinence diaper according to the invention,
Figure 4 shows a longitudinal section along the line IV-IV in Figure 3.

### DETAILED DESCRIPTION OF ILLUSTRATIVE EMBODIMENTS:

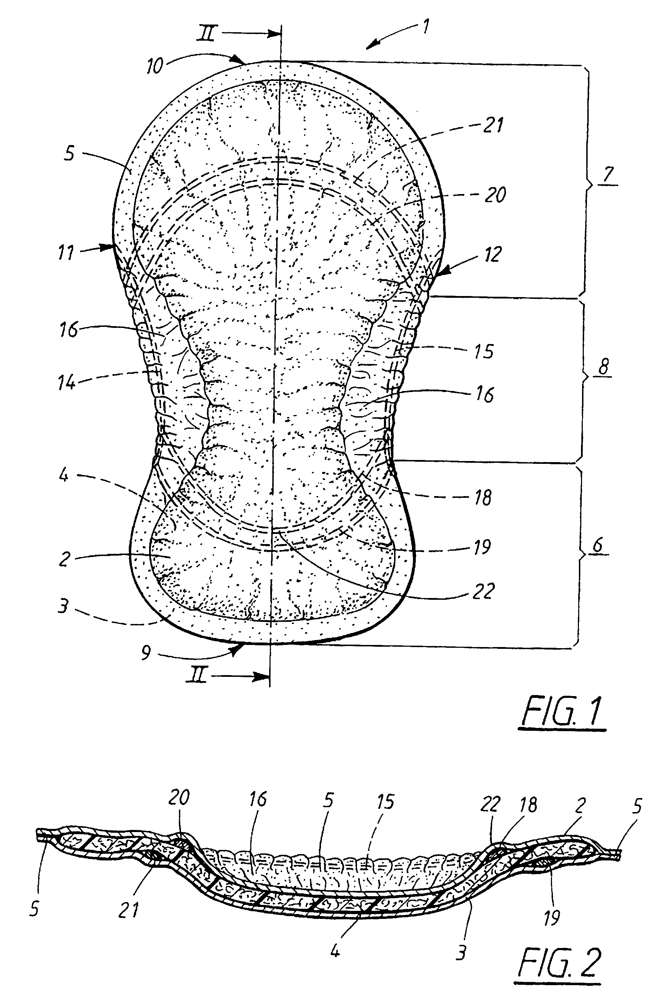

The incontinence diaper 1 shown in Figures 1 and 2 comprises a liquid-permeable cover sheet 2, a liquid-tight cover sheet 3, and an absorption body 4 enclosed between the two cover layers 2, 3. The cover sheets 2, 3 have a slightly greater extent in the diaper plane than does the absorption body 4 and they form a protruding, flexible cover edge 5 around the periphery of the absorption body. The cover sheets 2, 3 are also connected to each other within the cover edge, for example by gluing, or welding using heat or ultrasound. It is generally also advantageous if the cover sheets 2, 3 are secured to the absorption body 4, for example by gluing.

The liquid-permeable cover sheet 2 can be made of any material suitable for the purpose. Commonly occurring liquid-permeable cover sheets can be made from nonwoven material, perforated plastic film, cast, knitted or woven netting, or liquid-permeable foam. Both synthetic and natural materials can be used, such as laminates and various material combinations.

The liquid-tight cover sheet 3 is generally made of a thin plastic film, although other types of material are also possible. For example, dense nonwoven material or laminates of plastic film and nonwovens can be used. The liquid-tight cover sheet is expediently impenetrable to liquid, but it can also advantageously allow gas and vapour to pass through.

The absorption body 4 is also of a conventional type. Absorbent articles such as incontinence diapers often have an absorption body which is essentially made up of one or more layers of cellulose fluff pulp. It is also common to use polymer materials with a high liquid absorption capacity, often referred to as superabsorbents, for decreasing the weight and volume of the article, and for binding liquid in the article. A number of further types of absorption materials are available and can be used either alone or in combination with each other. For example, absorbent fibres other than cellulose fluff pulp can be used, such as absorbent foam material, turf or the like. In addition, the absorption body can comprise further components, for example tissue layers, wetness indicators, liquid-spreading layers, liquid-receiving layers, etc. It is also possible to treat the absorption body to influence its properties. For example, it is customary to provide the absorption body with compressions and to treat components in the absorption body with wetting agents.

The incontinence diaper 1 shown in Figures 1 and 2 has an asymmetrical hour-glass shape, with a longitudinal direction and a transverse direction. The incontinence diaper has a front portion 6, a rear portion 7 and a central portion 8 which is situated between the front portion 6 and the rear portion 7. The incontinence diaper 1 also has a front edge 9 and a rear edge 10 which run essentially in the transverse direction of the diaper, and two side edges 11, 12 which run essentially in the longitudinal direction of the diaper. The absorption body 4 has approximately the same shape as the diaper 1 as a whole, but it has a more pronounced hour-glass shape.

In order to curve the incontinence diaper in the longitudinal direction, elastic members 14, 15 are arranged along the side edges 11, 12 of the diaper, in the cover edge 5 extending beyond the absorption body. The elastic members 14, 15 also serve to raise the cover edge 5 at the central portion 8 of the diaper to form a liquid-tight edge barrier 16. Suitable elastic members for this purpose are, for example, bands or threads of elastic material such as rubber, polyurethane. Elastic film materials, elastic nonwoven materials, elastic nettings or elastic foam can be used. The elastic members 14, 15 are arranged along the side edges 11, 12 of the diaper with a certain prestressing. This can be achieved by the elastic members being stretched out before they are secured to the diaper. Another way of obtaining gathered elastic edges is to use material which gathers and becomes elastic only after it has been treated, for example by heating, wetting or irradiation. Such a material is secured to the diaper and activated either at a later stage in the manufacturing process or only when the diaper is in use.

According to the invention, the diaper shown in Figures 1 and 2 also has further sets of elastic members 18, 19, 20, 21 which are arranged with prestressing at the front portion 6 and rear portion 7, respectively, of the diaper.

The first set of elastic members comprises an inner elastic member 18 and an outer elastic member 19 which are arranged at the front portion 6 of the diaper and run in an arc shape from one side edge 11 across the absorption body 4 to the second side edge 12. The inner elastic member 18 is arranged between the absorption body 4 and the liquid-permeable cover sheet 2 and is secured to the liquid-permeable cover sheet 2, for example by gluing, welding or sewing. The inner elastic member 18 can also be secured to the absorption body 4 of the diaper 1 if so desired. The inner elastic member 18 runs in an arc directed towards the front edge 9 of the diaper. The forces which thereby act on the diaper draw the diaper together both in the longitudinal direction and in the transverse direction. The inner elastic member 18 thus has a certain shaping effect on the diaper, but mainly gives rise to formation of a curved, essentially transversely raised barrier 22. The raised barrier 22 comprises the inner elastic member 18 and a raised portion of the liquid-permeable cover sheet 2. In the case where the inner elastic member 18 is also secured to the absorption body 4 of the diaper, the raised barrier 22 will also comprise material from the absorption body 4.

The expression "arc directed towards the front edge of the diaper" means that the elastic runs along a convex path which bulges out in the direction towards the front edge of the diaper.

The outer elastic member 19 is arranged on that surface of the absorption body 4 which, during use of the incontinence diaper 1, is intended to face towards the user's underwear. The outer elastic member, as is shown in Figure 2, can be arranged between the liquid-tight cover sheet 3 and the absorption body 4. Alternatively, the outer elastic member 19 can be arranged on the outside of the liquid-tight cover sheet 3. In the last-mentioned design, there is the advantage that the outer elastic member 19 also serves as a friction-increasing element on contact between the outside of the liquid-tight cover sheet 2 and the user's underwear. The outer elastic member is secured to the liquid-tight cover sheet 3 and/or to the absorption body 4. The main aim of the outer elastic member 19 is to shape the diaper both in the longitudinal direction and the transverse direction and thereby to strengthen the shaping effect of the inner elastic member 18, so that a cup-shaped area is formed on that surface of the diaper 1 which is intended to face towards the user during use. In order to obtain this effect, it is necessary for the outer elastic member 19 to be arranged nearer the front edge 9 of the diaper than is the inner elastic member 18. In addition, the elastic members 18, 19 should be arranged at a mutual distance which is from 5 mm to 75 mm and preferably from 10 mm to 35 mm.

In a corresponding manner, the second set of elastic members 20, 21 is arranged at the rear portion 7 of the diaper, with an inner elastic member 20 secured to the liquid-permeable cover sheet 2 in an arc between the side edges 11, 12 of the diaper and an outer elastic member 21 secured to the liquid-tight cover sheet 3, in an arc between the inner elastic member 20 and the rear edge 10 of the diaper. The elastic members 20, 21 arranged in the rear portion should also be placed at a mutual distance of from 5 mm to 75 mm and preferably from 10 mm to 35 mm.

The elastic members 14, 15 along the side edges 11, 12 of the diaper and the inner elastic members 18, 20 in the front portion 6 and rear portion 7 of the diaper enclose a cup-shaped area on that surface of the diaper which is intended to receive the excreted body fluid during use. The elastic members 14, 15, 18, 20 form raised elastic barriers, or basin walls, around the cup-shaped area. The barriers, or the basin walls, prevent liquid from running freely across the surface of the article and out over the edges and during use they form a sealing edge bearing against the user's body.

All the elastic members included in the diaper can be of the different types indicated in the description of the elastic members 14, 15 arranged along the side edges 11, 12 of the diaper. The elastic members can be secured to the diaper by being glued, sewn or welded on. Each elastic member can comprise two or more part members, for example two or more threads or bands.

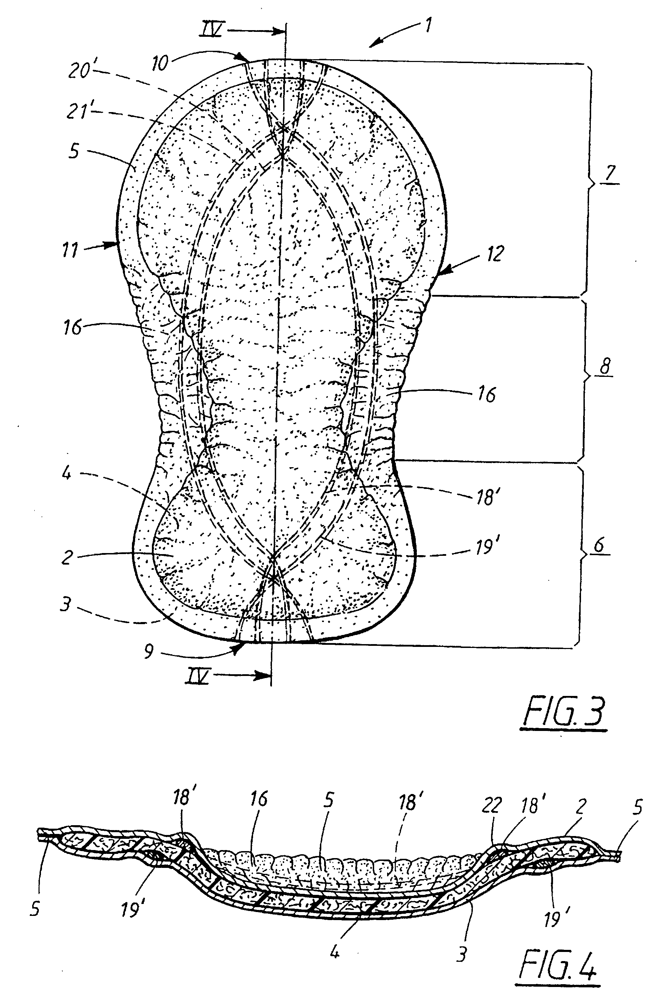

In the embodiment shown in Figures 3 and 4, those parts corresponding to similar components in the embodiment according to Figures 1 and 2 have been provided with the same reference numbers. These similar components are therefore not described in connection with the embodiment according to Figures 3 and 4.

The embodiment according to Figures 3 and 4 does not include the transverse elastic members 18, 19, 20 and 21 or the longitudinal elastic members 14, 15 serving as leg elastics.

In the embodiment according to Figures 3 and 4, the abovementioned elastic members have been replaced by longitudinal first and second elastic members 18' and 19' and longitudinal third and fourth elastic members 20' and 21'.

The longitudinal first member 18' is secured to the liquid-permeable cover sheet 2 and forms an arc-shaped curve which, for the greater part of its length, runs across the absorption body and, over a smaller part, in the crotch area of the article, runs outside the side edge of the absorption body. The longitudinal first elastic member 18' forms a first arc-shaped curve which is directed outwards towards the first side edge 12 of the article and which with its end portions crosses the longitudinal line of symmetry of the article. The statement that the longitudinal first elastic member forms a first arc-shaped curve which is directed outwards towards the first side edge 12 of the article signifies that the curve bulges convexly towards the said side edge.

A second longitudinal elastic member 19' is secured to the liquid-tight cover sheet 3 and forms a second arc-shaped curve, which is essentially uniform to the first longitudinal arc-shaped curve.

A third longitudinal elastic member 20' is arranged symmetrical with the first longitudinal elastic member 18' on the opposite side of the longitudinal centre line of the article and is secured to the liquid-permeable cover sheet 2.

In a corresponding manner, a fourth longitudinal elastic member 21' is secured to the liquid-tight cover sheet 3 and is arranged symmetrical with the second longitudinal elastic member 19' on the opposite side of the longitudinal centre line of the article.

The inner longitudinal elastic members 18', 20' can also be secured to the absorption body 4 of the article. These last-mentioned elastic members draw the diaper together both in the longitudinal direction and in the transverse direction and have a certain shaping effect on the diaper, but above all they give rise to the formation of a curved and raised barrier 22, as can be seen from Figure 4.

The curved longitudinal elastic members 18', 20' give rise to forces acting on the inner liquid-permeable cover sheet in the direction of the radius of curvature, as a result of which the said raised barrier 22 is formed. The raised barriers 22 can also comprise material from the absorption body 4.

The outer longitudinal elastic members 19' and 21' contribute to shaping the diaper both in the longitudinal direction and in the transverse direction and to increasing the shaping effect such that a cup-shaped area is formed on that surface of the diaper which during use is intended to face towards the user.

As in the embodiment according to Figures 1 and 2, the distance between the inner longitudinal elastic members 18' and 20' should be from 5 mm to 75 mm and preferably from 10 mm to 35 mm.

For manufacturing reasons, the embodiment according to Figures 3 and 4 is especially suitable when the diapers are made with their longitudinal direction in the machine direction.

Although the invention has been described above in connection with an incontinence diaper, it must be understood that the invention encompasses all types of absorbent articles which are intended to be placed inside the user's underwear and which do not have means for joining them together to the shape of pants. This category of absorbent articles includes not only incontinence diapers of different shapes and sizes, but also sanitary towels. The invention moreover covers those absorbent articles with transverse curved elastic members which do not have elastic members along the side edges, and articles which are only provided with the transverse elastic members at their end portion. An absorbent article according to the invention can also be provided with securing members, suitably in the form of one or more self-adhesive areas, by means of which the article can be secured inside the user's underwear. It is also possible to provide the article with some type of friction coating, such as friction adhesive or foamed rubber.

## Claims

1. Absorbent article having a longitudinal direction and a transverse direction, two side edges (11, 12) running in the longitudinal direction, and a first end edge (9) running in the transverse direction and a second end edge (10) running in the transverse direction, and comprising a liquid-tight cover sheet (3), a liquid-permeable cover sheet (2), an absorption body (4) enclosed between the two cover sheets (2, 3), and elastic members (18, 19) which run in an arc shape across the article between two opposite edges, **characterized in that** a first elastic member (18, 18') is secured to the liquid-permeable cover sheet (2) and forms a first arc-shaped curve which runs in across the absorption body and is directed towards a first edge (9, 12) of the article, and **in that** a second elastic member (19) is secured to the liquid-tight cover sheet (3) and forms a second arc-shaped curve which runs in across the absorption body and is directed towards said first edge (9,12), the second elastic member (19) being arranged between the first elastic member (18) and the said first edge (9, 12).

2. Absorbent article according to Claim 1, **characterized in that** the first arc-shaped curve and the second arc-shaped curve are uniform.

3. Absorbent article according to Claim 1 or 2, **characterized in that** the distance between the first elastic member (18) and the second elastic member (19) is from 5 millimetres to 75 millimetres, preferably from 10 millimetres to 35 millimetres.

4. Absorbent article according to Claim 1, 2 or 3, **characterized in that** the said first and second elastic members (18, 19) are arranged at the front portion (6) and run in the transverse direction of the article between the side edges (11, 12) of the article, the arc-shaped curve being directed towards a first end edge, **in that** a third elastic member (20) is secured to the liquid-permeable cover sheet (2) and forms a third arc-shaped curve which is directed towards the second end edge (10) of the article, and **in that** a fourth elastic member (21) is secured to the liquid-tight cover sheet (3) and forms a fourth arc-shaped curve, said third and fourth elastic members are arranged at the rear portion, the fourth elastic member (21) being arranged between the third elastic member (20) and the second end edge (10) of the article.

5. Absorbent article according to Claim 1, 2 or 3, **characterized in that** the said first and second elastic members (18', 19') run in the longitudinal direction of the article between the end edges (9, 10) of the article, the first and second arc-shaped curves being directed towards a first side edge (12), **in that** a third elastic member (20') is secured to the liquid-permeable cover sheet (2) and forms a third arc-shaped curve which is directed towards the second side edge (11) of the article, said third elastic member (20') is arranged symmetrical with the first longitudinal elastic member (18') on the opposite side of the longitudinal centre line of the article, and **in that** a fourth elastic member (21') is secured to the liquid-tight cover sheet (3) and forms a fourth arc-shaped curve, the fourth elastic member (21') being arranged between the third elastic member (20') and the second side edge (11) of the article.

6. Absorbent article according to Claim 4 or 5, **characterized in that** the third arc-shaped curve and the fourth arc-shaped curve are essentially uniform.

7. Absorbent article according to Claim 4, 5 or 6, **characterized in that** the distance between the third elastic member (20) and the fourth elastic member (21) is from 5 millimetres to 75 millimetres, preferably from 10 millimetres to 35 millimetres.

8. Absorbent article according to any of Claims 1 to 4, **characterized in that** elastic members (14, 15) are arranged along the side edges (11, 12) of the article.

9. Absorbent article according to any of the preceding claims, **characterized in that** the second elastic member (19, 19') is arranged on the outside of the liquid-tight cover sheet (3).

10. Absorbent article according to any of Claims 4 to 8, **characterized in that** the fourth elastic member (21, 21') is arranged on the outside of the liquid-tight cover sheet (3).

## Patentansprüche

1. Absorptionsartikel mit einer Längsrichtung und einer Querrichtung, zwei Seitenrändern (11, 12), die in der Längsrichtung verlaufen, und einem ersten Endrand (9), der in der Querrichtung verläuft und einem zweiten Endrand (10), der in der Querrichtung verläuft, ferner mit einer flüssigkeitsdichten Abdecklage (3), einer flüssigkeitsdurchlässigen Abdecklage (2), einem Absorptionskörper (4), der zwischen den beiden Abdecklagen (2, 3) eingeschlossen ist, und elastischen Elementen (18, 19), die in einer Bogenform über den Artikel zwischen zwei entgegengesetzten Rändern verlaufen, **dadurch gekennzeichnet, dass** ein erstes elastisches Element (18, 18') an die flüssigkeitsdurchlässige Abdecklage (2) befestigt ist und eine erste bogenförmige Krümmung bildet, die nach innen über den Absorptionskörper verläuft und zu einem ersten Rand (9, 12) des Artikels gerichtet ist, und dadurch, dass ein zweites elastisches Element (19) an die flüssigkeitsdichte Abdecklage (3) befestigt ist und eine zweite bogenförmige Krümmung bildet, die nach innen über den Absorptionskörper verläuft und zu dem ersten Rand (9, 12) gerichtet ist, wobei das zweite elastische Element (19) zwischen dem ersten elastischen Element (18) und dem ersten Rand (9, 12) angeordnet ist.

2. Absorptionsartikel nach Anspruch 1, **dadurch gekennzeichnet, dass** die erste bogenförmige Krümmung und die zweite bogenförmige Krümmung gleichmäßig sind.

3. Absorptionsartikel nach Anspruch 1 oder 2, **dadurch gekennzeichnet, dass** der Abstand zwischen dem ersten elastischen Element (18) und dem zweiten elastischen Element (19) von 5 Millimeter bis 75 Millimeter, vorzugsweise von 10 Millimeter bis 35 Millimeter beträgt.

4. Absorptionsartikel nach Anspruch 1, 2 oder 3, **dadurch gekennzeichnet, dass** das erste und zweite elastische Element (18, 19) an dem Vorderabschnitt (6) angeordnet sind und in der Querrichtung des Artikels zwischen den Seitenrändern (11, 12) des Artikels verlaufen, wobei die erste bogenförmige Krümmung zu einem ersten Endrand gerichtet ist, und dass ein drittes elastisches Element (20) an die flüssigskeitsdurchlässige Abdecklage (2) befestigt ist und eine dritte bogenförmige Krümmung bildet, die zu dem zweiten Endrand (10) des Artikels gerichtet ist, und dass ein viertes elastisches Element (21) an die flüssigkeitsdichte Abdecklage (3) befestigt ist und eine vierte bogenförmige Krümmung bildet, wobei das dritte und vierte elastische Element an dem hinteren Abschnitt angeordnet sind, wobei das vierte elastische Element (21) zwischen dem dritten elastischen Element (20) und dem zweiten Endrand (10) des Artikels angeordnet ist.

5. Absorptionsartikel nach Anspruch 1, 2 oder 3, **dadurch gekennzeichnet, dass** das erste und zweite elastische Element (18', 19') in der Längsrichtung des Artikels zwischen den Endrändern (9, 10) des Artikels verlaufen, wobei die erste und zweite bogenförmige Krümmung zu einem ersten Seitenrand (12) gerichtet sind, dass ein drittes elastisches Element (20') an die flüssigkeitsdurchlässige Abdecklage (2) befestigt ist und eine dritte bogenförmige Krümmung bildet, die zu dem zweiten Seitenrand (11) des Artikels gerichtet ist, wobei das dritte elastische Element (20') symmetrisch mit dem ersten längs-elastischen Element (18') an der entgegengesetzten Seite der Längsmittellinie des Artikels angeordnet ist, und dass ein viertes elastisches Element (21') an die flüssigkeitsdichte Abdecklage (3) befestigt ist und eine vierte bogenförmige Krümmung bildet, wobei das vierte elastische Element (21') zwischen dem dritten elastischen Element (20') und dem zweiten Seitenrand (11) des Artikels angeordnet ist.

6. Absorptionsartikel nach Anspruch 4 oder 5, **dadurch gekennzeichnet, dass** die dritte bogenförmige Krümmung und die vierte bogenförmige Krümmung im wesentlichen gleichmäßig sind.

7. Absorptionsartikel nach Anspruch 4, 5 oder 6, **dadurch gekennzeichnet, dass** der Abstand zwischen dem dritten elastischen Element (20) und dem vierten elastischen Element (21) von 5 Millimeter bis 75 Millimeter, vorzugsweise von 10 Millimeter bis 35 Millimeter beträgt.

8. Absorptionsartikel nach einem der Ansprüche 1 bis 4, **dadurch gekennzeichnet, dass** elastische Elemente (14, 15) entlang der Seitenränder (11, 12) des Artikels angeordnet sind.

9. Absorptionsartikel nach einem der vorangehenden Ansprüche, **dadurch gekennzeichnet, dass** das zweite elastische Element (19, 19') an der Außenseite der flüssigkeitsdichten Abdecklage (3) angeordnet ist.

10. Absorptionsartikel nach einem der Ansprüche 4 bis 8, **dadurch gekennzeichnet, dass** das vierte elastische Element (21, 21') an der Außenseite der flüssigkeitsdichten Abdecklage (3) angeordnet ist.

## Revendications

1. Article absorbant ayant une direction longitudinale et une direction transversale, deux bords latéraux (11, 12) orientés dans la direction longitudinale, et un premier bord d'extrémité (9) orienté dans la direction transversale et un deuxième bord d'extrémité (10) orienté dans la direction transversale, et comprenant une feuille d'enveloppe (3) imperméable aux liquides, une feuille d'enveloppe (2) perméable aux liquides, un corps absorbant (4) enfermé entre les deux feuilles d'enveloppe (2, 3), et des éléments élastiques (18, 19) qui sont placés en arc en travers de l'article entre deux bords opposés, **caractérisé en ce qu'**un premier élément élastique (18, 18') est fixé à la feuille d'enveloppe (2) perméable aux liquides et forme une première courbe en forme d'arc qui va d'un côté à l'autre du corps absorbant et est dirigé vers un premier bord (9, 12) de l'article, et **en ce qu'**un deuxième élément élastique (19) est fixé à la feuille d'enveloppe (3) imperméable aux liquides et forme une deuxième courbe en forme d'arc qui va d'un côté à l'autre du corps absorbant et est dirigé vers ledit premier bord (9, 12), le deuxième élément élastique (19) étant disposé entre le premier élément élastique (18) et ledit premier bord (9, 12).

2. Article absorbant selon la revendication 1, **caractérisé en ce que** la première courbe en forme d'arc et la deuxième courbe en forme d'arc sont identiques.

3. Article absorbant selon la revendication 1 ou 2, **caractérisé en ce que** la distance entre le premier élément élastique (18) et le deuxième élément élastique (19) est comprise entre 5 millimètres et 75 millimètres, de préférence entre 10 millimètres et 35 millimètres.

4. Article absorbant selon la revendication 1, 2 ou 3, **caractérisé en ce que** lesdits premier et deuxième éléments élastiques (18, 19) sont placés dans la partie avant (6) et sont orientés dans la direction transversale de l'article entre les bords latéraux (11, 12) de l'article, la courbe en forme d'arc étant dirigée vers un premier bord d'extrémité, **en ce qu'**un troisième élément élastique (20) est fixé à la feuille d'enveloppe (2) perméable aux liquides et forme une troisième courbe en forme d'arc qui est dirigée vers le deuxième bord d'extrémité (10) de l'article, et **en ce qu'**un quatrième élément élastique (21) est fixé à la feuille d'enveloppe (3) imperméable aux liquides et forme une quatrième courbe en forme d'arc, lesdits troisième et quatrième éléments élastiques sont placés dans la partie arrière, le quatrième élément élastique (21) étant placé entre le troisième élément élastique (20) et le deuxième bord d'extrémité (10) de l'article.

5. Article absorbant selon la revendication 1, 2 ou 3, **caractérisé en ce que** lesdits premier et deuxième éléments élastiques (18', 19') sont orientés dans la direction longitudinale de l'article entre les bords d'extrémité (9, 10) de l'article, les première et deuxième courbes en forme d'arc étant dirigées vers un premier bord latéral (12), **en ce qu'**un troisième élément élastique (20') est fixé à la feuille d'enveloppe (2) perméable aux liquides et forme une troisième courbe en forme d'arc qui est dirigée vers le deuxième bord latéral (11) de l'article, ledit troisième élément élastique (20') est placé symétriquement par rapport au premier élément élastique longitudinal (18') sur le côté opposé de l'axe longitudinal de l'article, et **en ce qu'**un quatrième élément élastique (21') est fixé à la feuille d'enveloppe (3) imperméable aux liquides et forme une quatrième courbe en forme d'arc, le quatrième élément élastique (21') étant placé entre le troisième élément élastique (20') et le deuxième bord latéral (11) de l'article.

6. Article absorbant selon la revendication 4 ou 5, **caractérisé en ce que** la troisième courbe en forme d'arc et la quatrième courbe en forme d'arc sont essentiellement identiques.

7. Article absorbant selon la revendication 4, 5 ou 6, **caractérisé en ce que** la distance entre le troisième élément élastique (20) et le quatrième élément élastique (21) est comprise entre 5 millimètres et 75 millimètres, de préférence entre 10 millimètres et 35 millimètres.

8. Article absorbant selon l'une quelconque des revendications 1 à 4, **caractérisé en ce que** des éléments élastiques (14, 15) sont placés le long des bords latéraux (11, 12) de l'article.

9. Article absorbant selon l'une quelconque des revendications précédentes, **caractérisé en ce que** le deuxième élément élastique (19, 19') est placé sur le côté extérieur de la feuille d'enveloppe (3) imperméable aux liquides.

10. Article absorbant selon l'une quelconque des revendications 4 à 8, **caractérisé en ce que** le quatrième élément élastique (21, 21') est placé sur le côté extérieur de la feuille d'enveloppe (3) imperméable aux liquides.
